# NEW EUROPEAN PATENT SPECIFICATION

(11) **EP 1 341 482 B2**
(45) Date of publication and mention of the opposition decision: **18.05.2016**
(45) Mention of the grant of the patent: 20.10.2010
(21) Application number: 01992086.7
(22) Date of filing: 11.12.2001
(51) Int. Cl.: A61F 2/915

(54) **STENT HAVING HELICAL ELEMENTS**
STENT MIT SPIRALEN ELEMENTEN
STENT A ELEMENTS EN HELICE

(30) Priority: 11.12.2000 US 254688 P
(43) Date of publication of application: 10.09.2003
(60) Divisional application: 10184477.7
(73) Proprietor: OrbusNeich Medical, Inc., Ft Lauderdale, FL 33309 (US)
(72) Inventor: ADDONIZIO, Scott, J., Fort Lauderdale, FL 33315 (US); CAMP, David, L., Jr., Hillsboro Beach, Fl 30062 (US); BECKER, Gary, J., Miami, FL 33156 (US); PAZIENZA, John, D., Pompano Beach, FL 33060 (US)
(74) Representative: Jilderda, Anne Ayolt
(86) International application number: PCT/US2001/048034
(87) International publication number: WO 2003/017870

(56) References cited:
- EP-A- 0 884 029
- EP-A- 0 968 689
- EP-A1- 0 876 806
- EP-A1- 0 983 753
- EP-B1- 2 311 412
- WO-A1-00/13611
- WO-A1-00/30563
- WO-A1-00/45742
- WO-A1-94/17754
- WO-A1-98/20810
- WO-A1-99/39660
- WO-A2-01/89421
- WO-A2-95/26695
- US-A- 6 132 460

## Description

### BACKGROUND OF THE INVENTION

### 1. Field of the Invention

The present invention relates to prosthetic stents. In particular, the present invention relates to stents having helical elements.

### 2. Description of Related Art

Stents are prosthetic devices that are implanted in the lumen of a vessel inside the body to provide support for the vessel's wall. Structural support from stents is particularly important in angioplasty procedures. Typically, stents are implanted within a vessel system to reinforce vessels that are partially occluded, collapsing, weakened, or abnormally dilated. More generally, stents can be used inside any physiological conduit or duct including, for example, arteries, veins, bile ducts, the urinary tract, alimentary tracts, the tracheobronchial tree, a cerebral aqueduct or the genitourinary system. Stents may be used in both humans and animals.

There are typically two types of stents: self expanding stents and balloon expandable stents. Self expanding stents automatically expand once they are released and assume a deployed, expanded state. A balloon expandable stent is expanded using an inflatable balloon catheter. The balloon is inflated to plastically deform the stent Balloon expandable stents may be implanted by mounting the stent in an unexpanded or crimped state on a balloon segment of a catheter. The catheter, after having the crimped stent placed thereon, is inserted through a puncture in a vessel wall and moved through the vessel until it is positioned in the portion of the vessel that is in need of repair. The stent is then expanded by inflating the balloon catheter against the inside wall of the vessel. Specificially, the stent is plastically deformed by inflating the balloon so that the diameter of the stent is increased and remains at an increased state. In some situations, the vessel in which the stent is implanted may be dilated by the stent itself when the stent is expanded.

The Palmaz-Schatz^{tm} stent, which is disclosed in the Handbook of Coronary Stents by Patrick W. Serruys et al. (Martin Dunitz, LTD 1998), is an example of a balloon expandable stent that had been implanted in hundreds of thousands of patients. The Palmaz-Schatz^{tm} stent, like other known stents, has certain limitations. These include, but are not limited to: (i) low stent-to-vessel ratio uniformity, (ii) comparative rigidity of the stent in a crimped as well as deployed state, and (iii) limited flexibility making delivery and placement in narrow vessels difficult Stent-to-vessel ratio generally refers to the degree that the vessel wall is supported by the stent in its expanded state and preferably should be uniform throughout the length of the stent. Furthermore because the Palmaz-Schatz^{tm} stent consists of one or more bridges that connect a number of consecutively slotted tubes, there are a number of bare areas in the vessel after the expansion of the stent These shortfalls arc common to many stents.

EP-A-0 884 029 discloses an expandable stent comprising a main body, wherein the main body has a generally cylindrical shape and a cylindrical axis and, when the stent is unexpanded, the main body comprises a plurality of expandable helical segments. The present invention provides a stent according to claim 1.

The present invention is directed to expandable stents that have relatively uniform stent-to-vessel ratios when expanded and other desirable properties. The stents of the present invention comprise a generally cylindrically shaped main body having a plurality of expandable helical segments. The main body is comprised of a plurality of cylindrical main body elements that are joined together by the helical segments. The cylindrical elements have cylindrical axes that are collinear with the cylindrical axis of the main body. The cylindrical elements are formed from a plurality of circumferential elements that are joined together by the expandable helical segments. In some embodiments, the stent may comprise endzones that straddle the main body.

For example, the stent may comprise a first non-helical endzone and a second non-helical endzone that straddle the main body. The main body is generally cylindrically shaped and has a cylindrical axis. A plurality of adjacent main body cylindrical elements are connected together to form the main body of the stent. Each main body cylindrical element is comprised of a plurality of expandable first and second circumferential elements. In some example, the second circumferential elements have a circumferential dimension less than the circumferential dimension of the first circumferential elements. Each second circumferential segment in each main body cylindrical element is connected to two first circumferential segments. In addition, each second circumferential segment in each main body cylindrical element is connected to a second circumferential segment in an adjoining main body cylindrical element thereby forming a plurality of helixes in the main body of the stent.

For example, the main body is comprised of a plurality of first helical segments each having a substantially identical first pitch and a plurality of second helical segments, each having a substantially identical second pitch. The first and second pitches are generally different. In at least one example, the second pitch is twice that of the first, and at least one first helical segment crosses one of the second helical segments.

The stents of the present invention may be manufactured from a tubular member by removing material from the tube to form a first endzone region, a second endzone region, and a middle region. By removing material from the middle region a plurality of parallel helical segments will remain and a plurality of circumferential segments will remain connecting the helical segments. Alternatively, the stent may be formed from a tube by removing material such that at least two sets of helical segments remain with each set having a different pitch.

### BRIEF DESCRIPTION OF THE DRAWINGS

Figure 1 is a three dimensional view of one embodiment of a stent according to the present invention in its unexpanded state.
Figure 2 is planar view of a flattened portion of the circumference of the stent in Figure 1.
Figure 3 is an enlarged portion of Figure 2.
Figure 4 is another planar view of a flattened portion of the circumference of a stent according to the present invention in its unexpanded state.
Figure 5 is an enlarged view of a portion of Figure 4 showing a first circumferential element of the stent.
Figure 6 is an enlarged view of a portion of Figure 4 showing a second circumferential element of the stent.
Figure 7 is a planar view of a flattened portion of the stent in Figure 1 showing a plurality of sets of helical segments propagating through the stent's body.
Figure 8 is a planar view of a flattened endzone that may be employed in a stent of the present invention.
Figure 9 is a planar view of a flattened portion of part of the endzone shown in Figure 8.

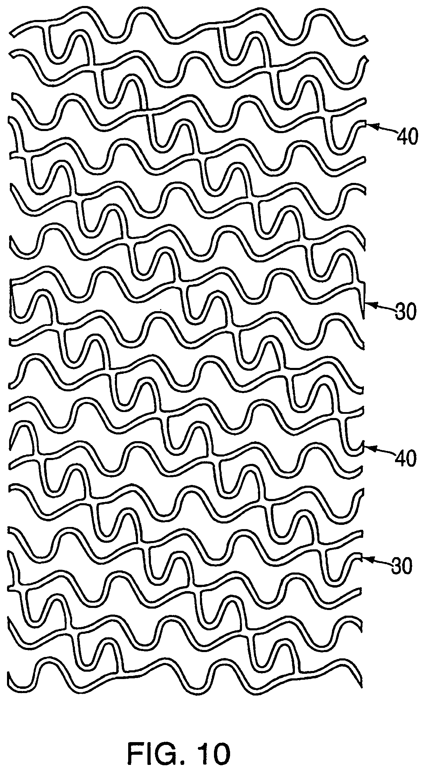

Figure 10 is a planar view of a flattened portion of an expandable stent according to the prior art, after the stent has been deployed in a lumen.
Figure 11 is three dimensional view of an alternative embodiment of the present invention.
Figure 12 is a three dimensional view of a stent.
Figure 13 is a planar view of the stent shown in 12.
Figure 14 is a detailed view of a portion of Figure 13.
Figure 15 is a detailed view of another portion of Figure 13.

### DETAILED DESCRIPTION OF THE INVENTION

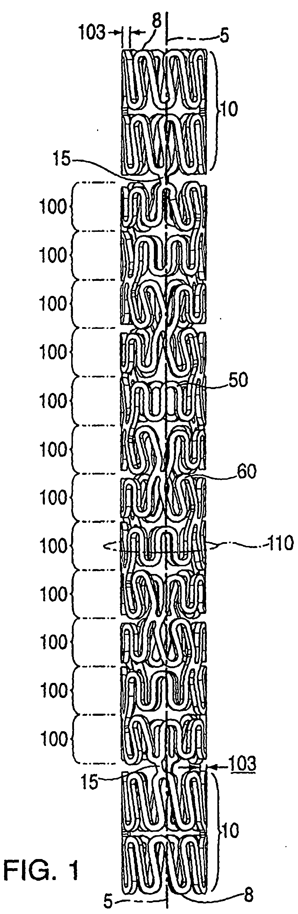

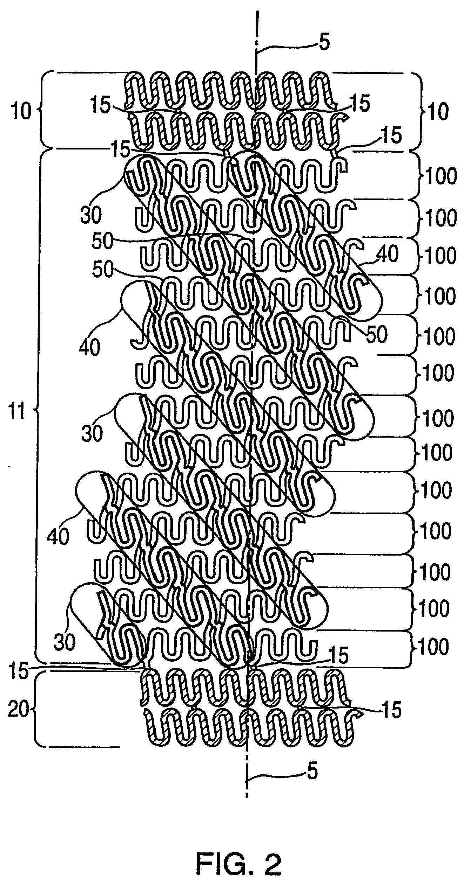

The present invention is directed to an expandable stent. In one embodiment, as is shown in Figures 1 and 2, the stent comprises a generally cylindrical shaped main body section **11** having a cylindrical axis **5** and a wall thickness **103.** The wall thickness **103** may optionally be uniform throughout the stent. The main body section **11** is comprised of a plurality of helical segments **30** and **40** and a plurality of main body cylindrical elements **100,** each having cylindrical axes (not shown) that are collinear with the main body cylindrical axis **5**. The main body cylindrical elements **100** are each comprised of circumferential elements **50** that are joined together by the helical segments **30** and **40** to form individual cylinders **100**.

The stent may also have a first endzone **10** and a second endzone **20** that straddle the body section **11**. In some example, such as the one shown in Figure 1, the endzones **10** and **20** may advantageously provide the stent with square outer edges **8.** The stent may be manufactured from stainless steel, or other suitable materials. In most embodiments, it is desirable that the material, or a portion of the material, be radiopaque and that the various segments that form the stent be contiguous. Although, in some embodiments, the various segments that make up the stent can be distinct elements that are joined together.

The main body **11,** shown in Figures 1 and 2, may be formed in numerous ways. The body **11** contains two or more first helical segment **30** and **40** that are generally parallel to each other and an opposite each other by 180.° In general, the first helical segments **30** and **40** will be spaced equidistant along the circumference **110** of the main body **11.** The first helical segments **30** and **40** are joined by a plurality of circumferential segments **50** to form a plurality main body cylindrical elements **100,** which are generally cylindrically shaped. The circumferential segments **50** make up a majority of the circumference **110** of each cylindrical element **100.** In addition to joining the circumferential elements **50** to form cylindrical elements **100,** the helical segments **30** and **40** connect each cylindrical element **100** to an adjacent cylindrical element **100** to form the main body **11**.

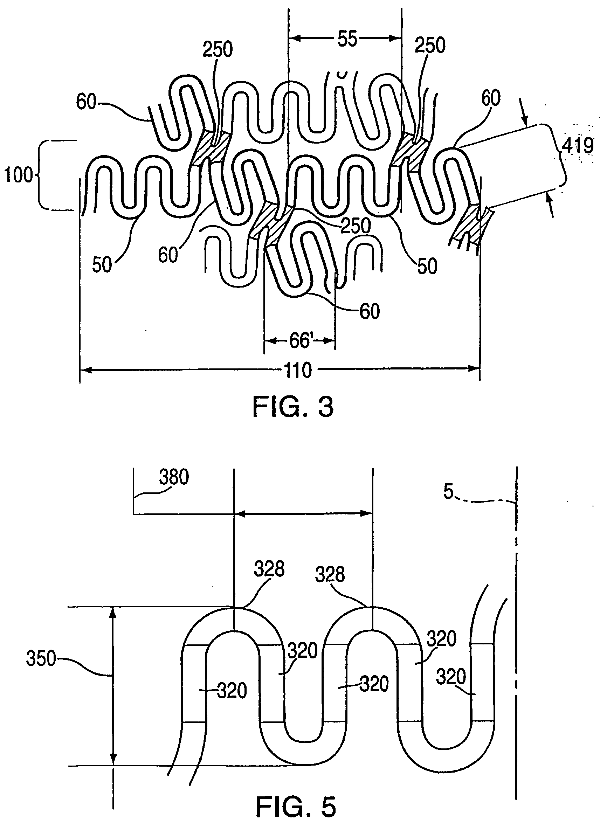

As is shown in Figure 3, the body of the stent **11** comprises a plurality of main body cylindrical elements **100** formed from first circumferential segments **50** that are joined with second circumferential segments **60.** The second circumferential segments **60** of each cylindrical element **100** are joined with second circum-ferential segments **60** of adjacent cylindrical elements **100** to form a plurality of first helical segments **30** and **40** in the main body **11.** (See Fig. 2). Each first circumferential segment **50** may have a circumferential dimension **55** and each second circumferential segments **60** may have a circumferential dimension **66.** (See Fig.3) In some examples, it may be desirable for the circumferential dimension **55** of the first expandable element **50** to be larger than the circumferential dimension **66** of the second expandable element **60.**

The first circumferential segment **50** is an expandable segment formed from plurality of segments joined together to form a repeating pattern. The pattern, such as the one shown in the figures 1-3, may be a repeating pattern that resembles a square wave form having curved peaks and valleys. Other repeating patterns, may be used that enable the segment to expand when a radial force is exerted on the stent from the inside or collapse radially when an external crimping force is applied.

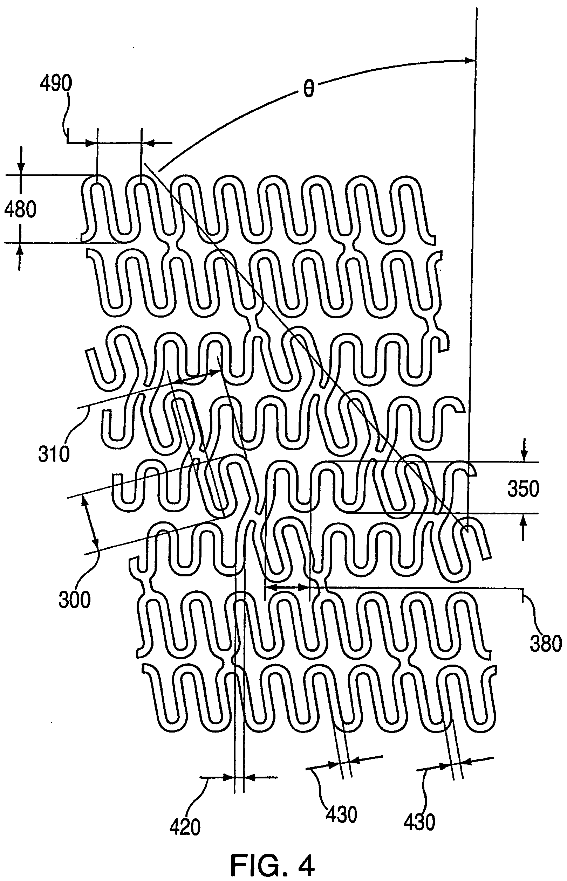

The first circumferential elements **50** may have a filament width **420** (see Fig. 4). In one example, the filament width may vary between 0.05 mm (0.002 inches) and 0.18 mm (0.007 inches), but is preferably about 0.13 mm (0.0050 inches). Other filament widths may be used depending on the parameters of the stent.

In the embodiment shown in Figs. 1-5, the first circumferential elements **50** comprise linear portions **320** and curved portions **328** that join the linear portions **320** together to form a repeating pattern. In some, but not all, embodiments, the linear portion **320** may be parallel to the cylindrical axis of the stent. The first circumferential segment **50** has an amplitude **350** and a period **380.** In one example the amplitude may range from 0.5 mm to 2.0 mm and the period may range from 0.5 mm to 2.0 mm. In some embodiments, the amplitude is less than the period. Other amplitudes and periods may be used depending on the overall stent design and performance constraints.

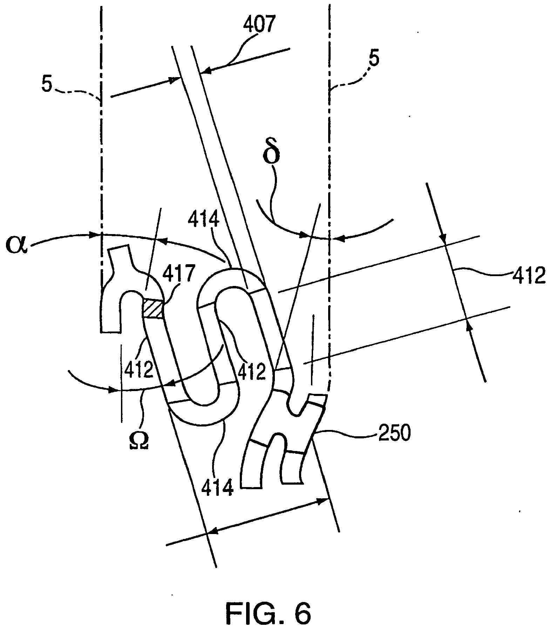

The second circumferential elements **60** are joined together in a helical pattern to form one of two helical segments **30** or **40**. In the embodiment shown in Fig 6, the second circumferential element **60** comprises linear portions **412** and curved portions **414** having a filament width **407,** and resembles generally an S-shaped structure. In addition, the second element circumferential segment **60** may have an angled portion **417** attached to the linear portion **412** at an end opposite that of the curved portion **414.** The angled portion may be oriented to form an angle α relative to the cylindrical axis of the stent **5** in range of 0-45 degrees. In at least one example, the preferable angle α is about 10 degrees. In some embodiments, the linear portions **412** of the second circumferential element **60** lies at an angle Ω relative to the cylindrical axis of the stent, wherein Ω preferably ranges from 0 to 45 degrees. When viewed in a planar fashion as in Fig. 2, the linear portions **412** may, in some embodiments, form an angle Ω, relative to the cylindrical axis of the stent in some examples, Ω may be approximately equal to the helical angle of the first helical segments **30** and **40.** In one embodiment, the second circumferential elements **60** may have an amplitude **300** (see Figs. 3, 4, and 6) ranging from 0.5 mm to 2.0 mm and a period ranging from 0.5 mm to 2.0 mm. Other ranges may be used depending on the particular stent size and design being employed. In one example, the preferred period is about 0.82 mm and the preferred length of the linear portion **412** is about 0.5 mm and the amplitude **300** is about 0.38 mm. The amplitude of the second circumferential element **60** may be greater than, equal to, or less than the amplitude of the first circumferential element **50**. In one example, the circumferential contributions of the first circumferential elements **50** to the overall circumference of the main body **11** is greater than the circumferential contribution of the second circumferential element **60**, in terms of either circumferential length or circumferential cylindrical surface area. In one example, the stent may have an overall outer surface area of about 0.029 square inches.

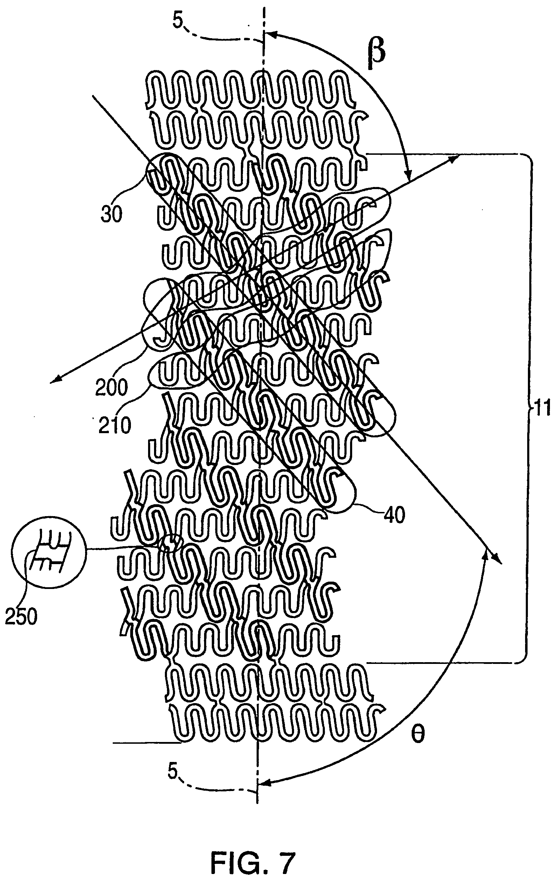

As is shown in Figure 7, the stent has a main body **11** comprised of two first helical segments **30** and **40,** as well as two second helical segments **200** and **210.** The first and second helical segments **30, 40** and **200, 210,** respectively, are joined together to form a generally cylindrically shaped body **11.** The first and second helical segments share a common connecting element **250.** In some examples, the common connecting element **250** may be H-shaped and the two generally parallel linear portions of the H-shaped connecting segment **250** may form an angle δ relative to the axis 5. (See Fig. 6). δ may, in one example, be about 14 degrees. As is shown in Figure 7, the first helical segments **30** and **40** and second helical segments **200** and **210** may have different pitches, i.e. number of spirals per unit length, which results in the first and second helical segments as having different helical angles (θ and β, respectively) i.e. the angle of the helical segment relative to the cylindrical axis 5 of the stent. In one example, the second helical segments **200** and **210** have a pitch approximately twice that of the first helical segments. In one example θ may vary from 0 to 45 degrees and is preferably about 40 degrees and P is preferably about twice θ. In other embodiments the angle θ may range from 0 to 90 degrees to the circumference **110** of each cylindrical element **100.**

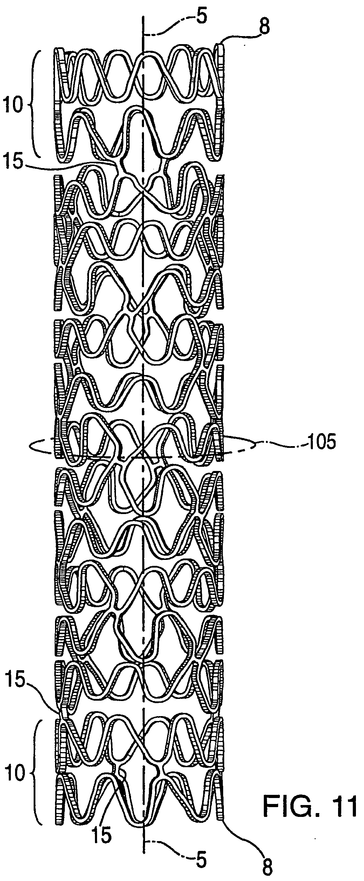

As is shown in Figures 2, 3, 4, and 6, the helical segments **30, 40** are circumferentially expandable (i.e. they expand along the circumference of the stent) and are formed from a plurality of circumferential elements **60** that in turn are made up of linear **412** and/or curved **414** segments (see Fig. 6) that each have a filament width **407** (see Fig. 6) that is less than the circumferential dimension **66** of the circumferential element **60** (see Fig. 3). In some example, each helical segment **30** or **40** will make a total contribution to the circumference of each cylindrical element **100** that is greater than the filament width **407.** The circumferential contribution of each helical segment **30** or **40** to the overall circumference of the stent **(110** in Figure 1 or **105** in Figure 11) may be greater than the circumferential contribution of the filament widths **407** of the segments (e.g. **412** and **414)** making up the circumferential elements **60** that in turn make up the helical segments. (i.e., in some examples the circumferential contribution of the helical segments **30** and **40** to the circumference **110** of each cylindrical element **100** is more than just a function of the filament width **407,** e.g., it may be a function of the geometry of the element **60.)** For the example shown in Figures **1** and **11****,** this is the case when the stent is in both the unexpanded and expanded state. The geometry of the helical segments **30** and **40** are a factor in determining their expandability.

Likewise, the helical segments **200, 210** are circumferentially expandable and are comprised of other circumferential elements **50** that are in turn comprised of linear **320** and/or curved segments **328** (see Figs. 3 and 5) that have a filament width **420** (see Fig. 4). The contribution of the helical segments **200, 210** to the over-all circumferential dimension **110** of each cylindrical element **100** is greater than just the contribution of the filament widths **420** of the individual segments **320** and **328** that make up the elements **50** that in turn make up the helical segments **200, 210.** The geometry of the elements **50** making up the helical segments **200, 210** may be a more important factor in determining the circumferential contribution of the helical segments **200** and **210** to the overall stent circumference than the filament width **420.** Thus, in one embodiment of the present invention, the circumference of the stent **110** in its unexpanded state and the circumference **105** when the stent is expanded are primarily functions of the geometry of the elements **50** and **60** that make up the helical segments **30, 40** and **200**, **210,** respectively.

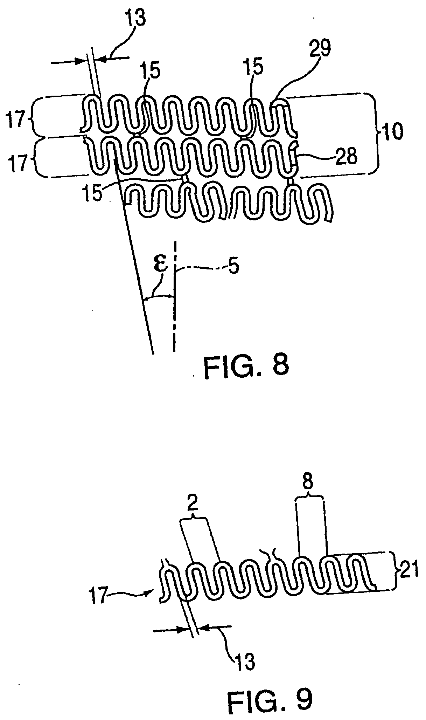

Some, but not all examples, of the present invention may employ endzones **10** and **20.** (See Figs. 1, 2, and 11). Stents that employ endzones will generally have two endzone regions straddling a central zone in the middle of the stent. The stents may also have a transition region between the endzone and the central zone. The transition region serves to help smoothly transition between the expanded middle region and an portions of the end of the stent that remain unexpanded after the stent is implanted. The size and characteristics of the transition region are a function of the material and geometry of the stent. For example, the transition range properties vary as a function of, among other things, the helical angle of the first helical segments, the number of curved segments located in the endzones, and the angle ε of the linear portions of the segments forming the endzones. (See e.g. Fig. 8).

The endzones **10** and **20** may take numerous form. In some examples, the endzones may be comprised of one or more rings **17.** (See Fig. 8). The rings **17** may be generally cylindrically shaped, and in some embodiments, right cylindrically shaped. In one embodiment, the rings are formed from linear segments **28** joined together by curved segments **29** to form a pattern. The pattern, which is preferably - but not necessarily - a repeating pattern may take numerous forms, including the one shown. The endzones **10** and **20** may be comprised of a plurality of rings **17** attached together. Struts **15** may be used to attach the rings together to form the endzone and to attach the endzone to the main body **11**. The struts, in some examples, act as cantilever springs and there stiffness, which is a function of their width and thickness, may define bending properties of the stent along its cylindrical axis **5**.

In the example shown in Figs. 1, 7, 8, and 9, which is exemplary only, the linear segments **28** in the endzone **10,** are oriented at an angle ε relative to the cylindrical axis of the stent. The angle ε may range from 0 to 45 degrees and in one embodiment is preferably about 10 degrees. The segments of the endzone may have a filament width **13** of between 0.05 and 0.18 mm (0.002 and 0.007 inches). In one embodiment, the repeating pattern of the endzone has a period 2 of about 0.68 mm (0.027 inches) and an amplitude **21** of about 1.59 mm (0.043 inches) Other values may be used. As is shown in Figure 1, the struts **15,** which are but one way to attach the endzones **10** and **20** to the main body **11,** may, in one embodiment have a width of between 0.05 mm (0.002 inches) and 2.03 mm (0.08 inches) and preferably the width does not exceed the wall thickness, which typically -but not necessarily ranges from about 0.05 to 0.02 mm (0.002 to 0.008 inches).

The stent of the present invention may, after insertion into a vessel, be expanded such that it plastically deforms from the unexpanded state to an expanded state having a diameter increase of about 400 to 500%, which results in a larger circumference **105.** (See Figure 11). Figure 11 depicts the stent shown in Figure 1 in an expanded state. Upon expansion the stent's outer diameter in one particular embodiment increases from 1.0 mm to 3.00 mm and maintains a stent-to-vessel ratio in the expanded state that is greater than on average 16%.

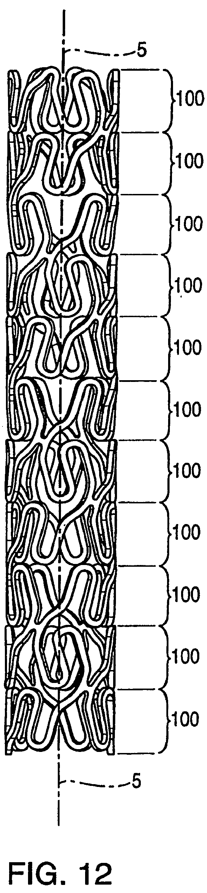

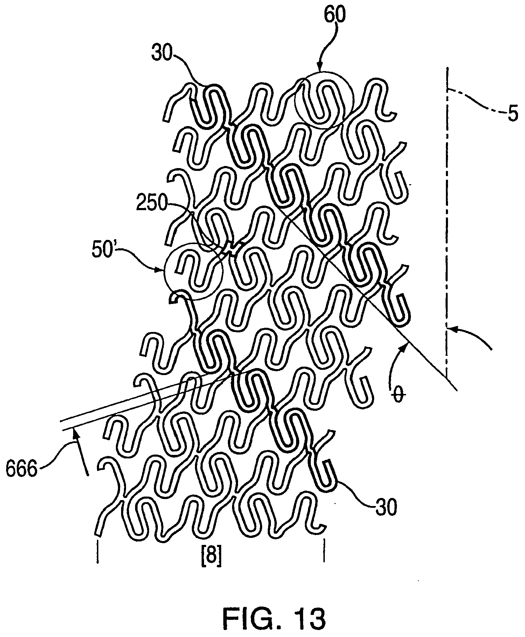

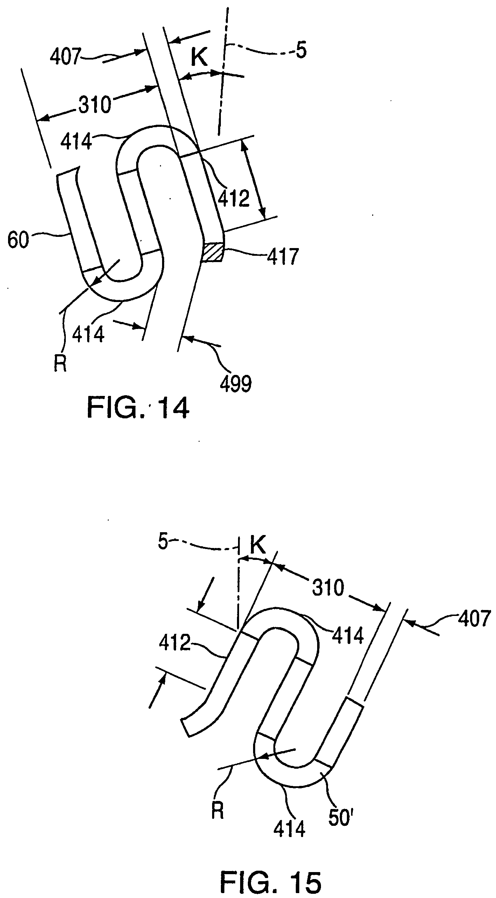

While endzones **10** and **20** may be used to provide square edge, not all stents according to the present invention require endzones. Figures 12-15 depict an endzoneless stent Like the stent shown in Figures 1-9, the stent of Figures 12-15, comprises a plurality of adjacent cylindrical elements **100.** The cylindrical elements **100** are formed from a plurality of first circumferential elements **50'** and second circumferential elements **60.** The first circumferential elements **50'** of the stent in Figures 12-15 are substantially identical to the second circumferential element **60** except that they are rotated to have a different orientation. The circumferential elements may be generally S-shaped having a linear portion **412,** a curved portion **414** having a radius **R,** and an angled portion **417. R** may-vary widely depending on overall stent characteristics and in one embodiment varies between 0.02 and 0.5 mm (0.001 and 0.02 inches) and is preferably about 0.21 mm (0.0083 inches). The angled portion **417** is spaced a distance **499** from the linear portion. In one particular example, the distance **499** may vary from 0.002 to 0.020 inches and is preferably about 0.007 inches. The filament width **407** of the elements may, in one embodiment, be about 0.13 mm.

Adjacent cylindrical elements **100** are joined together by connecting first circumferential elements **50'** in each cylindrical element **100** with first circumferential elements **50'** in an adjacent cylindrical element **100,** such that the first circumferential elements **50'** in adjacent cylindrical elements **100** form helixes through the stent and such that second circumferential elements form helixes through the stent having an angle θ relative to the axis **5**. In some embodiments, a connecting segment **250** (see Fig. 7) is used to connect first circumferential elements in adjacent cylindrical elements **100** and to connect second circumferential elements **60** in adjacent cylindrical elements **100.** In addition, the connecting segment, connects first circumferential elements **50**' in each cylindrical element **100** with two second circumferential elements **60** in each cylindrical element **100.** In one example, the individual cylindrical elements **100** are adjacent to each other and are located a distance **666** apart. In one embodiment, the preferred may range between 0.05 and 0.5 mm (0.002 and 0.020 inches), and is preferably about 0.009 inches.

Various modifications may be made to the stent to change its overall characteristics without deviating from the scope of the invention as defined by the claim

The stent of the present invention may be manufactured in numerous ways. The stent may be formed from a metallic tube by removing various portions of the tube's wall to form the patterns described herein. The resulting stent will thus be formed from a single contiguous piece of material, eliminating the need for connecting various segments together. Material from the tube wall maybe removed using various techniques including laser (YAG laser for example), electrical discharge, chemical etching, metal cutting, a combination of these techniques, or other well known techniques. See e.g. U.S Patent Nos. 5,879,381 to Moriuchi et al. and 6,117,165 to Becker. Forming stents in this manner allows for creation of a substantially stress-free structure where the helical segments are integral with the circumferential elements. In one example, the tube from which the stent is formed may have an internal diameter of about 3.0 mm, a wall thickness of about 1.0 mm and a length of about 30 mm. Tubes having other dimensions may be used. In particular, the length may be adapted to that of the diseased part of the lumen in which the stent is to be placed. This may avoid using separate stents to cover the total diseased area.

## Claims

1. A balloon expandable stent comprising a main body (11), wherein the main body has a generally cylindrical shape and cylindrical axis (5) and, when the stent is unexpanded, the main body comprises a plurality of expandable helical segments (30, 40), wherein the main body further comprises a plurality of main body cylindrical elements (100) having collinear cylindrical axes, wherein the main body cylindrical elements (100) are adjacent to one another and attached to one another by the helical segments (30, 40), each main body cylindrical element (100) having a circumference (110) that is substantially identical to that of an adjacent cylindrical element, and comprising a plurality of expandable circumferential segments (50, 60) positioned between consecutive connecting elements (250) which connect said cylindrical element to an adjacent cylindrical element, wherein the circumferential segments (50, 60) are joined together by portions of the helical segments (30, 40) to form the cylindrical elements (100), wherein the plurality of circumferential segments (50, 60) comprise a majority of the circumference (110) of each cylindrical element (100), **characterized in that** the cylindrical elements (100) comprise first circumferential segments (50) alternating with second circumferential segments (60), **in that** said second circumferential segments resemble a generally S-shaped structure, having three linear portions connected to each other by two curved portions, **in that** said first circumferential segments have five linear portions connected to each other by four curved portions, **in that** adjacent cylindrical elements are connected to one another by two connecting elements (250), **in that** second circumferential segments (60) of adjacent cylindrical elements are joined together by connecting elements (250) to form one of two first expandable helical segments (30, 40), **in that** first circumferential segments (50) of adjacent cylindrical elements are joined together by connecting elements (250) to form one of two second expandable helical segments (200, 210), **in that** said first expandable helical segments (30, 40) are generally parallel one to another and 180 degrees apart, and **in that** said second expandable helical segments (200, 210) are generally parallel one to another and 180 degrees apart, and wherein the first circumferential element (50) comprise linear portions (320) and curved portions (328) that join the linear portions (320) together to form a repeating pattern, and wherein second helical segments cross the first helical segments in shared connecting elements.

## Patentansprüche

1. Expandierbarer Ballonstent, umfassend einen Hauptkörper (11) von im Wesentlichen zylindrischer Gestalt und mit zylindrischer Achse (5) und wobei, bei expandiertem Stent, der Hauptkörper eine Mehrzahl expandierbarer Spiralsegmente (30, 40) umfasst, wobei der Hauptkörper weiterhin eine Mehrzahl zylindrischer Hauptkörper-Elemente (100) umfasst mit koaxialen zylindrischen Achsen, wobei die zylindrischen Hauptkörperelemente (100) einander benachbart und durch spiralige Segmente (30, 40) aneinander befestigt sind, wobei jedes zylindrische Hauptkörperelement (100) einen Umfang (110) aufweist, der im Wesentlichen gleich jenem eines benachbarten zylindrischen Elementes ist, und umfassend eine Mehrzahl expandierbarer Umfangssegmente (50, 60), angeordnet zwischen aufeinanderfolgenden Verbindungselementen (250), die das zylindrische Element mit einem benachbarten zylindrischen Element verbindet, wobei die Umfangssegmente (50, 60) durch Teile der spiraligen Segmente (30, 40) miteinander verbunden sind, um die zylindrischen Elemente (100) zu bilden, wobei die Mehrzahl der Umfangssegmente (50, 60) einen Hauptteil des Umfanges (110) eines jeden zylindrischen Elementes (100) umfasst, **dadurch gekennzeichnet, dass** die zylindrischen Elemente (100) erste Umfangssegmente (50) umfassen, abwechselnd mit zweiten Umfangssegmenten (60), dass die zweiten Umfangssegmente im Wesentlichen S-förmige Struktur aufweisen mit drei linearen Teilen, die durch gekrümmte Teile miteinander verbunden sind, dass die ersten Umfangssegmente fünf Linearteile umfassen, verbunden miteinander durch vier gekrümmte Teile, dass einander benachbarte zylindrische Elemente miteinander verbunden sind durch zwei Anschlusselemente (250), dass die zweiten Umfangssegmente (60) einander benachbarter zylindrischer Elemente miteinander verbunden sind durch Anschlusselemente (250) zum Bilden eines von zwei ersten expandierbaren Spiralsegmenten (30, 40), dass die ersten Umfangssegmente (50) einander benachbarter zylindrischer Elemente miteinander verbunden sind durch Anschlusselemente (250) zum Bilden eines von zwei zweiten expandierbaren Spiralsegmenten (200, 210), dass die ersten expandierbaren Spiralsegmente (30, 40) im Wesentlichen parallel zueinander und 180° voneinander liegen, und dass die zweiten expandierbaren Spiralsegmente (200, 210) im Wesentlichen parallel zueinander und um 180° voneinander liegen, wobei die ersten Umfangselemente (50) lineare Teile (320) und gekrümmte Teile (328) umfassen, die die linearen Teile (320) miteinander verbinden, um ein Wiederholmuster zu bilden, und wobei die zweiten spiraligen Elemente die ersten spiraligen Elemente in anteiligen Verbindungselementen kreuzen.

## Revendications

1. Un stent expansible par ballonnet comprenant un corps principal (11), dans lequel le corps principal possède une forme généralement cylindrique et un axe cylindrique (5) et, lorsque le stent est non expansé, le corps principal comprend une pluralité de segments hélicoïdaux expansibles (30, 40), dans lequel le corps principal comprend également une pluralité d'éléments cylindriques de corps principal (100) possédant des axes cylindriques colinéaires, dans lequel les éléments cylindriques du corps principal (100) sont placés les uns à côté des autres et fixés les uns aux autres par les segments hélicoïdaux (30, 40), chaque élément cylindrique du corps principal (100) possédant une circonférence (110) qui est substantiellement identique à celle d'un élément cylindrique adjacent, et comprenant une pluralité de segments circonférentiels expansibles (50, 60) positionnés entre des éléments de raccordement consécutifs (250) qui raccordent ledit élément cylindrique à un élément cylindrique adjacent, dans lequel les segments circonférentiels (50, 60) sont reliés entre eux par des portions des segments hélicoïdaux (30, 40) pour former les éléments cylindriques (100), dans lequel les différents segments circonférentiels (50, 60) comprennent la majeure partie de la circonférence (110) de chaque élément cylindrique (100), **caractérisé en ce que** les éléments cylindriques (100) comprennent de premiers segments circonférentiels (50) alternant avec des seconds segments circonférentiels (60), **en ce que** lesdits seconds segments circonférentiels ressemblent à une structure généralement en forme de S, possédant trois portions linéaires raccordées les unes aux autres par deux portions incurvées, **en ce que** lesdits premiers segments circonférentiels possèdent cinq portions linéaires raccordées les unes aux autres par quatre portions incurvées, **en ce que** les éléments cylindriques adjacents sont reliés les uns aux autres par deux éléments de raccordement (250), **en ce que** les seconds segments circonférentiels (60) des élément cylindriques adjacents sont reliés ensemble par des éléments de raccordement (250) pour former l'un des deux premiers segments hélicoïdaux expansibles (30, 40), **en ce que** les premiers segments circonférentiels (50) des éléments cylindriques adjacents sont reliés ensemble par des éléments de raccordement (250) pour former l'un des deux seconds segments hélicoïdaux expansibles (200, 210), **en ce que** lesdits premiers segments hélicoïdaux expansibles (30, 40) sont généralement parallèles les uns aux autres et positionnés à 180 degrés les uns des autres, et **en ce que** lesdits seconds segments hélicoïdaux expansibles (200, 210) sont généralement parallèles les uns aux autres et positionnés à 180 degrés les uns des autres, et dans lequel les premiers éléments circonférentiels (50) comprennent des portions linéaires (320) et des portions incurvées (328) qui relient les portions linéaires (320) ensemble pour former un motif qui se répète, et dans lequel les seconds segments hélicoïdaux croisent les premiers segments hélicoïdaux en des éléments de raccordement communs.
